# EUROPEAN PATENT APPLICATION

(11) **EP 2 058 745 A2**
(43) Date of publication of application: **13.05.2009**
(21) Application number: 08253053.6
(22) Date of filing: 17.09.2008
(51) Int. Cl.: G06F 19/00

(54) **Context platform framework for aggregation, analysis and use of contextual information**

(30) Priority: 20.09.2007 US 858872
(71) Applicant: Intel Corporation, Santa Clara, CA 95054 (US)
(72) Inventor: Hatalkar, Atul N., Chandler, Arizona 85224 (US); Memmott, James L., Draper, Utah 84020 (US); Sandage, David A., Oregon 97116 (US)
(74) Representative: Beresford, Keith Denis Lewis

(57) **Abstract**

A context platform framework for aggregation, analysis and use of contextual information is described. An embodiment of a system includes an aggregator to receive context information from a plurality of providers via a provider application program interface (API), where the aggregator to aggregate the received context information. The system also includes an analyzer to analyze the received context information and the aggregated context information to generate computed context information. Then, based on the aggregated context information, the computed context information and one or more analysis rules, at least one of the aggregator and the analyzer to trigger an action for at least one client of a plurality of clients, where communication with the plurality of clients is accomplished via a client API. Other embodiments are described and claimed.

## Description

### BACKGROUND

Context aware systems or devices are concerned with acquisition of context (e.g., using sensors to perceive a situation), understanding of the context (e.g., matching a perceived sensory stimulus to a context), and application behavior based on the recognized context (e.g., triggering actions based on context).

Goals of context aware devices are to adapt their behaviors, make decisions, or take actions on behalf of a user without requiring explicit user inputs. For example, a context aware mobile phone may know that it is currently in the meeting room, and that the user has sat down. The phone may conclude that the user is currently in a meeting and automatically reject any unimportant phone calls.

In recent days, a variety of new sensors have become available in the marketplace. A corresponding set of new applications and usages are becoming available as well. Unfortunately, today's implementations of context aware devices show a narrow utilization of sensors, wherein specific sensors lead to specific usages. Such solutions do not allow the sensors to be used broadly.

### BRIEF DESCRIPTION OF THE DRAWINGS

Figure 1 illustrates one embodiment of a context platform framework for aggregation, analysis and use of contextual information.

Figure 2 illustrates one embodiment of a context engine.

Figure 3 illustrates one embodiment of a policy engine.

Figure 4 illustrates one embodiment of dependencies among logical components of the context engine.

Figure 5 illustrates one embodiment of a context schema.

Figure 6 illustrates one embodiment of an environment of the invention.

Figure 7 illustrates one embodiment of a logic flow illustrating how context information may be provided to the aggregator.

Figure 8 illustrates one embodiment of a logic flow illustrating how a client may access context data in the aggregator.

Figure 9 illustrates one embodiment of a logic flow illustrating how a client may provide an analysis rule to the analyzer.

Figure 10 illustrates one embodiment of a logic flow of the operation of the policy engine.

### DETAILED DESCRIPTION

Embodiments of the present invention provide a context platform framework for aggregation, analysis and use of contextual information. In embodiments, a context engine hides sensor-specific peculiarities from context clients and offers a unified mechanism for clients to access disparate context sources. In embodiments, sensors may include both physical and soft sensors (e.g., software based sources). An aggregator of the context engine decouples sources of the context information from the consumers of the context information (i.e. the clients) through aggregation of the context information. In embodiments, an analyzer of the context engine may conduct analysis on the aggregated context information to compute new context information of more useful nature, as perceived by the clients. As such, embodiments of the context engine facilitate out-of-band processing of the context information that is otherwise performed by the clients. Embodiments of the invention free context clients from the continuous tasks of context information acquisition, aggregation, monitoring and analysis and allow the clients to focus on their primary tasks or even enter power conserving sleep-states as needed. In embodiments, the clients may be triggered when "contextually significant" events or situations occur, as may be customized by the clients via analysis rules utilized by an analyzer of the context engine. A policy engine may trigger tangible actions either inside the physical platform that the context platform framework is implemented or in its surroundings based on changes in the aggregated context information, changes in the computed context information and control policy rules. In embodiments, context information is stored and processed by the context platform framework according to a context schema. Other embodiments may be described and claimed.

Various embodiments may comprise one or more elements or components. An element may comprise any structure arranged to perform certain operations. Each element may be implemented as hardware, software, or any combination thereof, as desired for a given set of design parameters or performance constraints. Although an embodiment may be described with a limited number of elements in a certain topology by way of example, the embodiment may include more or less elements in alternate topologies as desired for a given implementation. It is worthy to note that any reference to "one embodiment" or "an embodiment" means that a particular feature, structure, or characteristic described in connection with the embodiment is included in at least one embodiment. The appearances of the phrase "in one embodiment" in various places in the specification are not necessarily all referring to the same embodiment.

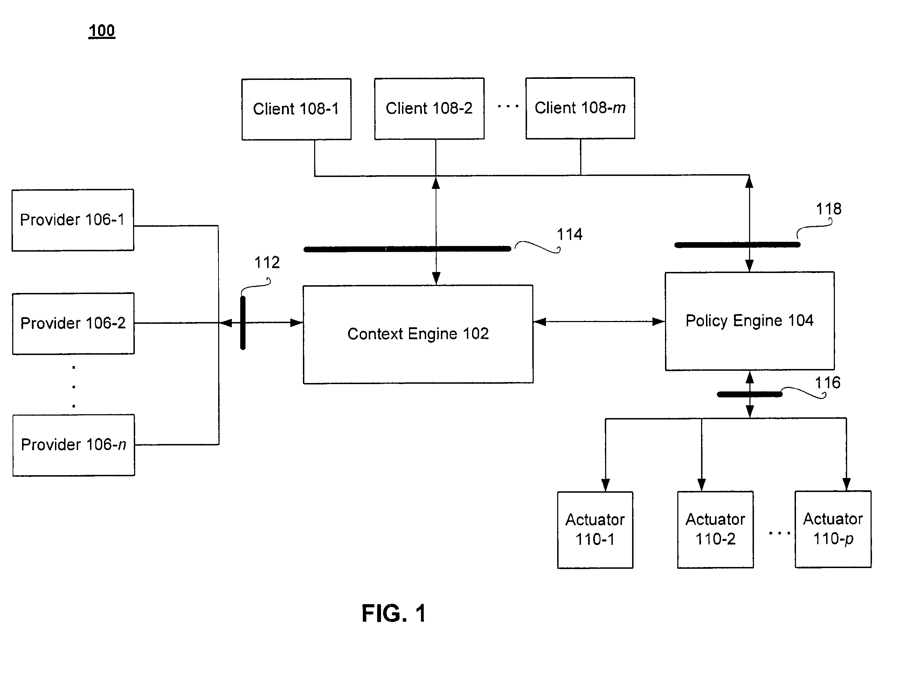

Figure 1 illustrates one embodiment of a context platform framework 100 for aggregation, analysis and use of contextual information. In one embodiment, context platform framework 100 comprises a context engine 102 and a policy engine 104. Context platform framework 100 also comprises one or more providers 106-1-*n*, where *n* is any positive integer; one or more clients 108-1-*m*, where *m* is any positive integer; and one or more actuators 110-1-*p*, where *p* is any positive integer.

In embodiments, the components of context platform framework 100 are 'usage agnostic.' Although both providers 106 and clients 108 are typically usage domain dependent, the design and implementation of framework 100 do not make any assumptions about the usage domain(s) in which framework 100 will be used.

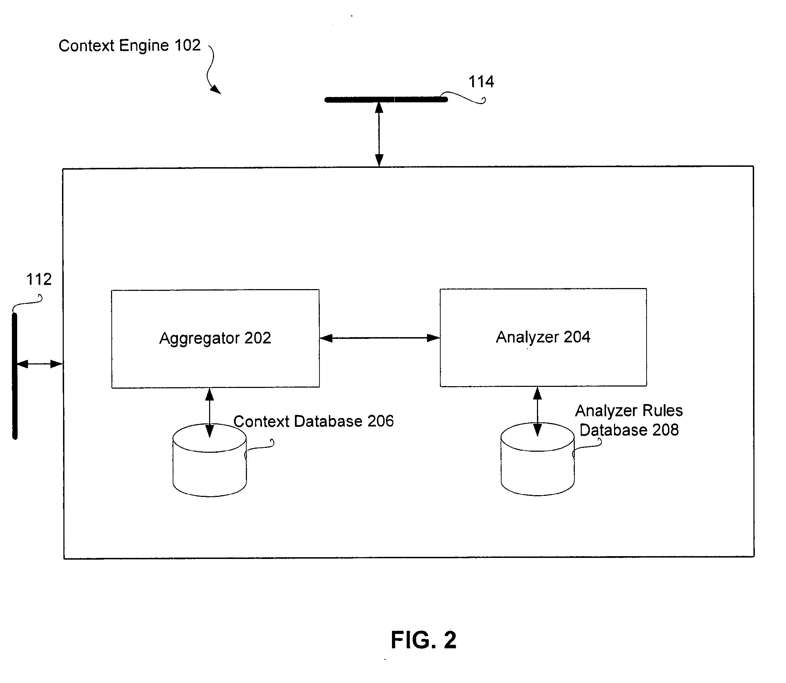

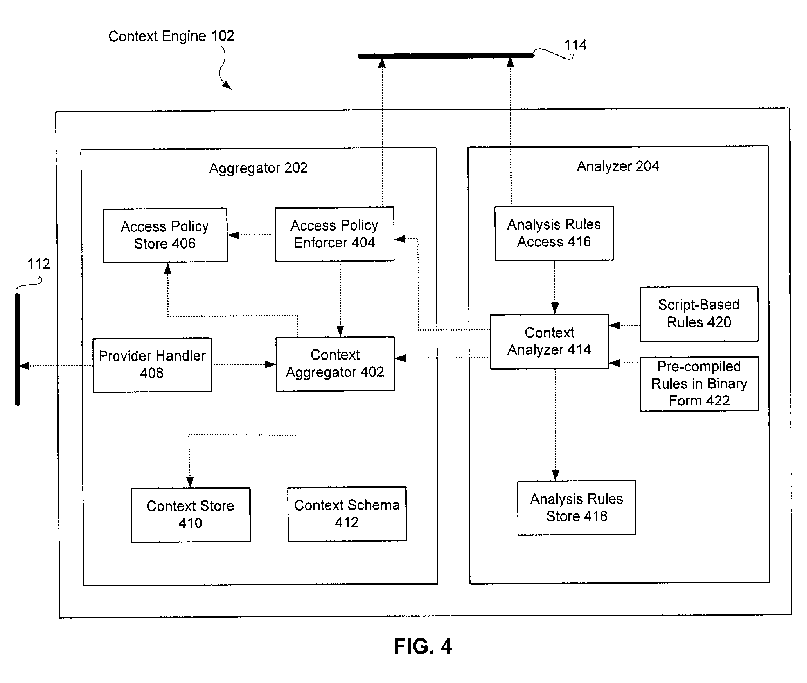

At a high level and in an embodiment, context data originating from physical sensors or software-based sources are continuously received by context engine 102 via providers 106. Providers 106 send the context data to context engine 102 via a provider application program interface (API) 112. Context engine 102 acquires, processes and maintains context information. Context engine 102 decouples the sources of the context information from clients 108. Context engine 102 may use the received data as "context" and make it available to clients 108. In embodiments, context engine 102 may also conduct analysis on the context information to generate additional context information of a more useful nature for clients 108 than the original context information received via providers 106. Clients 108 may supply customized analysis rules that are stored and utilized by context engine 102. Context engine 102 and clients 108 communicate via a context client API 114. Clients 108 may also use a policy API 118 to specify or adjust platform-behavior policies utilized by policy engine 104. Context engine 102 is described in more detail below with reference to Figures 2 and 4.

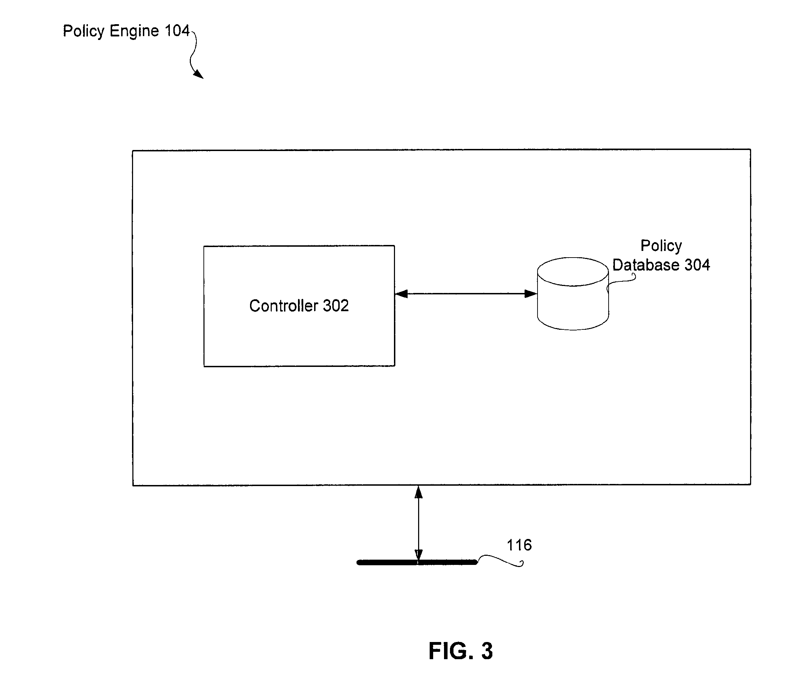

Context engine 102 also communicates with policy engine 104. Policy engine 104 may be viewed as a specialized client of context engine 102. Policy engine 104 triggers tangible actions either inside the physical platform that context platform framework 100 is implemented or in its surroundings based on changes in the aggregated context information, changes in the computed context information and control policy rules. Clients 108 may provide customized control policy rules that are stored and utilized by policy engine 104. Policy engine 104 is described in more detail below with reference to Figure 3. Policy engine 104 sends control commands and data to actuators 110 via an actuator API 116. Actuators 110 are capable of controlling hardware and software components within the device and in its surroundings.

In embodiments, context information is stored and processed by context platform framework 100 according to a context schema. The context schema consists of classes and associations, which represent fundamental elements of the context information. The schema classes form the basis of the processing actions that occur in context engine 102. The main classes of the context schema include context identifiers, context items, context groups and context data.

Context identifiers of the schema represent a particular type of context information (e.g., 'temperature' type). Context engine 102 may maintain and process multiple types of context information at the same time. Clients 108 may request context items by specifying a context identifier object.

Context items of the schema each represent a sample of the context information corresponding to a context identifier (e.g., a temperature reading). Typically, context engine 102 will maintain multiple context item objects for a particular context identifier. Context client API 114 enables clients 108 to access context item objects from context engine 102.

Context groups of the schema represent a collection of one or more context identifiers. Clients 108 may request a set of context items by specifying a context group obj ect.

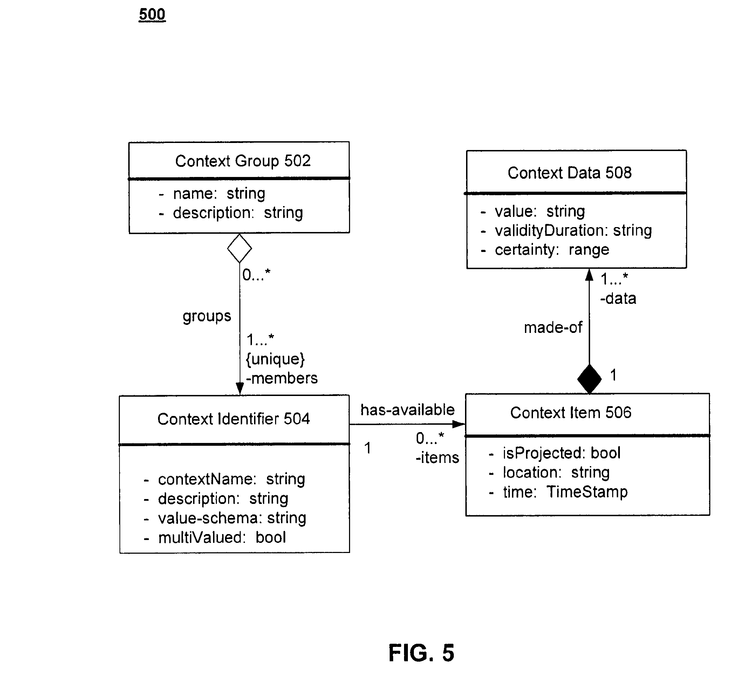

Context data of the schema represent one value of a context item. In some cases, a context item is allowed to have multiple valid values at the same time. Therefore, value representation is abstracted out in this class. These and other possible classes in the schema are described in more detail below with reference to Figure 5. Each of the elements or components in Figure 1 is described next in more detail.

In various embodiments, context platform framework 100 may be implemented as a wireless system, a wired system, or a combination of both. When implemented as a wireless system, framework 100 may include components and interfaces suitable for communicating over a wireless shared media, such as one or more antennas, transmitters, receivers, transceivers, amplifiers, filters, control logic, and so forth. An example of wireless shared media may include portions of a wireless spectrum, such as the RF spectrum and so forth. When implemented as a wired system, framework 100 may include components and interfaces suitable for communicating over wired communications media, such as input/output (I/O) adapters, physical connectors to connect the I/O adapter with a corresponding wired communications medium, a network interface card (NIC), disc controller, video controller, audio controller, and so forth. Examples of wired communications media may include a wire, cable, metal leads, printed circuit board (PCB), backplane, switch fabric, semiconductor material, twisted-pair wire, co-axial cable, fiber optics, and so forth.

In embodiments, providers 106 may be any type of entity (software, hardware or combination hardware/software) capable of collecting and providing data to context engine 102 via provider API 112 to be used as "context." Real-time data may be continuously collected via providers 106. Providers 106 may also be adapted to store real-time data via integrated long term storage, such as flash memory for example, and then transmit the data to context engine 102 at a later time. The integrated long term storage helps to ensure that no collected data are lost if there is no connection currently available with engine 102.

By way of example and not limitation, providers 106 may incorporate sensors of a physical nature such as platform sensors (e.g., battery charge, active radios, etc.); environmental sensors (e.g., location information via Global Positioning System (GPS) technology, humidity, nearby hazards, nearby devices, etc.); physiological sensors; and so forth. Physiological sensors may include one or more of an ECG to measure a broad array of cardiovascular characteristics (e.g., heart rate variability, ECG amplitude, ST segment analysis, QT interval, etc.); a pulse oximeter unit to measure oxygenation level; a multiaxial accelerometer to measure activity level and orientation; a temperature sensor to measure temperature level; a unit to measure galvanic skin response; a pulse wave velocity monitor to monitor blood pressure; a minimally invasive or noninvasive glucometry monitor unit to measure blood sugar; a unit to measure spirometry; a unit to measure respiration rate; a unit to measure speech characteristics (including volume, characteristics of speech associated with stress, speech cadence, latency, pause length, phonetic distinctness, word transposition, speech prosody, and indications of affect and variability in these speech characteristics); a unit to measure typing speed and errors; a unit to measure hormonal levels (e.g., an EMG device); a unit to measure caloric expenditure; a unit to measure temperature levels; a unit to measure muscle tension (e.g., cortisol); and so forth.

By way of example and not limitation, providers 106 may incorporate means of obtaining data from various "soft sources" (e.g. software sources). In embodiments, the software sources may include the sensing of stored context information in applications (e.g., user's next appointment, needed radio-type, desired display resolution, etc.); the sensing of dynamic context information such as in operating systems (e.g., memory usage, CPU load, etc.); the sensing of context information available in entities such as the Internet (e.g., traffic conditions, weather conditions, shopping, etc.); the sensing of user preferences (e.g., usage-history, etc.); and so forth.

In embodiments, providers 106 may have one or more of the followings responsibilities: hide the peculiarities of sensors and their interfacing details from context engine 102; when necessary, process raw sensor data to extract useful context information; provide context information to context engine 102 when requested; use an event notification mechanism to send context information to context engine 102 in asynchronous fashion; put sensors and related system components in low power mode when corresponding context-identifiers are not in use; adhere to provider API 112, as specified by context engine 102; and so forth.

Context engine 102 exposes a set of interfaces to manage its interactions with providers 106 and related external components (such as a provider installer). This set of interfaces is cumulatively referred to herein as provider API 112. In embodiments, provider API 112 may support one or more of the following functionalities: dynamically attach a new provider to context engine 102; dynamically detach an existing provider from the context engine 102; transfer detailed information of one or more context identifiers from a provider to context engine 102; facilitate synchronous calls from context engine 102 (e.g., calls requesting context item objects); facilitate notifications from providers 106 to context engine 102 (e.g., notifications indicating that an updated context item corresponding to a context identifier is available); facilitate commands from context engine 102 to providers 106 (e.g., command requesting a provider to enable/disable sourcing of context items for one or more context identifiers); and so forth.

In embodiments, provider API 112 allows providers 106 to specify context access rules. The context access rules may restrict access to certain context information to a certain class of clients 108. Context engine 102 enforces the context access rules. This mechanism ensures that clients 108 can gain access to a piece of context information that is either publicly available or authorized for access by a particular client or by a class of clients. In embodiments, the access restrictions are domain-specific and may be either pre-configured in a device or dynamically specified or edited via the context access rules. This is an example of how context platform framework 100 is modular and its components are dynamically loadable.

By way of example and not limitation, assume that a client is an application in a hospital that stores and provides access to patient information. Such information may include patient billing information, current medical conditions for the patient, and historical or past medical information for the patient. Here, the providers may provide context access rules to engine 102 regarding what types of hospital personnel may have access to the different types of patient information. For example, one context access rule may limit hospital administrators to patient billing information. Another context access rule may limit the nurses for the patient to the patient's current medical information, whereas another context access rule may allow doctors for the patient to have access to the patient's current and historical medical information.

In embodiments, clients 108 may be any application (with or without a user interface) that can benefit from context information. Clients 108 interact with context engine 102 via context client API 114. Clients 108 are responsible for adhering to client API 114 when interacting with context engine 102. Clients 108 may also be other software components, such as operating systems, platform utilities, platform tools, middleware libraries, "intermediate" software modules that use context information to offer value-added services to other modules, etc. In embodiments, majority of clients 108 are typically domain-specific.

Clients 108 may access context information generated via context engine 102 in a variety of ways via client API 114. For example, clients 108 may access context information either via synchronous and/or asynchronous events. With synchronous events, clients 108 request and receive information when needed from context engine 102. With asynchronous events, clients 108 request that updated context information be sent when available, or at the fulfillment of certain conditions. Here, context engine 102 may send notification events to appropriate clients.

Context engine 102 exposes a set of interfaces to manage its interactions with clients 108 and related external components. This set of interfaces is cumulatively referred to herein as context client API 114. In embodiments, context client API 114 may support one or more of the following functionalities: check availability of one or more context-identifiers; define new context groups by specifying their member context identifiers; access details about context identifiers and context groups; access context items corresponding to a context identifier; access context items corresponding to a context group; access context-items corresponding to a time and/or a location; specify and edit analysis rules; and so forth.

In embodiments, context client API 114 facilitates client authentication and controlled access to the context information. This mechanism helps to ensure that clients 108 can only gain access to a context item that is either publicly available to all clients or authorized for access by a particular client or a class of clients. In embodiments, access policies utilized by context engine 102 specify access permissions at the level of context identifiers and also for individual context items.

In embodiments, clients 108 may also use policy API 118 to specify or adjust platform-behavior policies utilized by policy engine 104. Clients 108 are responsible for adhering to policy API 118 when interacting with policy engine 104. In embodiments, policy API 118 may support the functionality of specifying and editing platform policies in policy engine 104.

Actuators 110 are capable of controlling hardware and software components within context platform framework 100 and its surroundings. By way of example and not limitation, actuators 110 may control hardware components to operate aspects of a context aware device, to change the resolution of a computer screen, to operate appliances (e.g., a thermostat, a light fixture, etc.), to switch between user-interface modalities, to page an on call doctor, and so forth.

Context platform framework 100 exposes a set of interfaces for the interactions between policy engine 104 and actuators 110. This set of interfaces is cumulatively referred to herein as actuator API 116.

Provider API 112, client API 114, actuator API 116 and policy API 118 may all be implemented via various mechanisms. In embodiments, these various mechanisms may include one or more of standard naive languages (such as C++), managed languages (such as Java) and protocol-based mechanisms such as web services.

Referring now to Figure 2, an embodiment of context engine 102 is illustrated. Context engine 102 acquires, processes and maintains context information. Context engine 102 may include an aggregator 202, an analyzer 204, a context database 206 and an analyzer rules database 208. Each of these components is described next in more detail.

In embodiments, aggregator 202 may facilitate the following tasks: maintain context information received from various sources (e.g., providers 106 and analyzer 204); enforce access policies associated with the context information; supply context information to clients 108; supply context information to analyzer 204; manage groups of the context identifiers which are specified by the clients; and so forth. In embodiments, an executing instance of context engine 102 may include up to one instance of aggregator 202.

Aggregator 202 receives context information from providers 106. Aggregator 202 can also receive context-information that is computed by analyzer 204. Aggregator 202 collects and stores the context information in context database 206. Aggregator 202 or context database 206 may use a context caching mechanism to allow aggregator 202 to quickly retrieve data. The context caching criteria may be of various types. For example, context caching criteria may include one or more of cache the last item accessed, cache latest n-items, cache items that were received during last 1-hour, and so forth. One or more of the context caching criteria may be in use at the same time. Aggregator 202 decouples the sources of the context information from clients 108.

In embodiments, aggregator 202 associates a timestamp and location-stamp references to all context-item objects when they are received. The clients 108 may access specific context-items, via context client API 114, by specifying the timestamp and/or location filters. This mechanism results in time-shifting and location-shifting modes of context access. Here, clients 108 may request context information applicable to a different time and/or location (e.g., the weather forecast for a particular city three months from now).

In embodiments, context group definitions used by aggregator 202 are usually domain-specific. The context group definitions may be established at the time of the build of the context aware device. Other embodiments allow clients 108 to specify and modify the context group definitions dynamically at run-time. Clients 108 may access context information stored as an individual item or as an aggregate of multiple items.

As stated above, some embodiments allow clients 108 to specify and modify the context group definitions dynamically at run-time. An example, not meant to limit the invention, may involve multiple context identifiers of the context schema stored in context database 206. These example context identifiers may each relate to a condition of the weather including "brightness condition", "humidity factor", and so forth. Client 108 may define a context item group and call it "weather". Here, the customized context item group of "weather" may include all of the context identifiers stored in context database 206 that include the related weather data such as "brightness condition", "humidity factor" and so forth.

In embodiments, analyzer 204 may facilitate the following tasks: receive and maintain analysis rules under the control of clients 108; monitor context information that is maintained in aggregator 202; execute analysis rules when input conditions specific to a context access rule are encountered; execute an analysis rule when aggregator 202 requests it; obtain required context information from aggregator 202 before executing an analysis rule; send new context information computed by the analysis rule to aggregator 202; and so forth.

In embodiments, an executing instance of context engine 102 may include up to one instance of analyzer 204. In embodiments, analyzer 204 may be always active or it may be dynamically loaded when one of the clients 108 requires the analyzer functionality. When analyzer 204 is dynamically loaded, then all necessary connections associated with analyzer 204 are also dynamically activated. Analyzer 204 depends in part on aggregator 202 and the interfaces between the two allow for dynamic loading and unloading of analyzer 204. This is one example of how context platform framework 100 is modular and its components are dynamically loadable.

In embodiments, analyzer 204 may conduct analysis on the context information to compute new context information of a more useful nature for clients 108 than the original context information received from providers 106. The analysis may take several forms, such as thresholding, pattern-detection, inferencing, signal processing, historical correlation, and so forth. Accordingly, analyzer 204 (and context engine 102 in general) may act as a "smart engine" and may include one or more aspects of artificial intelligence (AI) technologies to help facilitate the analysis of the context information.

In embodiments, analyzer 204 utilizes analysis rules supplied by the clients 108 to conduct analysis on the context information to generate additional context information. The analysis algorithms may be supplied by a device configurator at the build-time, for example, or by clients 108 at run-time. The analysis algorithms may be stored in analyzer rules database 208 and its associated cache. The computed context information is sent to the aggregator 202 for storage and further access by the clients 108 or analyzer 204.

By way of example and not limitation, analyzer 204 may support two modes of analysis algorithms. One mode of analysis algorithm may include script based analysis rules, as is well known in expert systems. Another mode of analysis algorithm may include pre-compiled rules supplied in binary form. In embodiments, analysis rules are typically usage domain dependent.

Embodiments of context engine 102 provide for out-of-band analysis of context information by having analyzer 204 perform the analysis that is typically performed by clients 108. Clients 108 are freed of the continuous tasks of context information acquisition, aggregation, monitoring and analysis. This allows clients 108 to focus on their primary tasks or even enter sleep-states as needed. In embodiments, clients 108 are triggered only when "contextually significant" events or situations occur, as may be customized by clients 108 via analysis rules utilized by analyzer 204. Embodiments of dependencies among logical components of aggregator 202 and analyzer 204 will be described below with reference to Figure 4.

As described above, context engine 102 also communicates with policy engine 104. Referring to Figure 3, an embodiment of policy engine 104 is illustrated. Policy engine 104 may include a controller 302 and a policy database 304. Controller 302 is responsible for triggering tangible actions inside the physical platform that context platform framework 100 is implemented and/or in its surroundings based on the aggregated context information, the computed context information and control policy rules stored in policy database 304. Controller 302 sends control commands and data to actuators 110 via actuator API 116. Actuators 110 are capable of controlling hardware and software components inside the physical platform that context platform framework 100 is implemented and its surroundings.

As discussed above, clients 108 may customize many aspects of the context information processed by context engine 102 to meet specific domain requirements. For example, clients 108 may provide one or more control policy rules to policy engine 104 that are relevant to a particular application. These customized control policy rules may be incorporated into a control policy rule set.

In embodiments, policy engine 104 may act as a "smart engine" and include artificial intelligence (AI) functionality to help facilitate the triggering of tangible actions inside the physical platform that context platform framework 100 is implemented and/or in its surroundings based on the aggregated context information, the computed context information and control policy rules stored in policy database 304.

In embodiments, policy engine 104 may be always active or it may be dynamically loaded when its functionality is required. When policy engine 104 is dynamically loaded, then all necessary connections associated with policy engine 104 are also dynamically activated. This is an example of how context platform framework 100 is modular and its components are dynamically loadable.

Figure 4 illustrates one embodiment of dependency among logical components or packages of context engine 102. In an embodiment, the word "dependency" is used as defined by the Unified Modeling Language (UML). Referring to Figure 4, aggregator 202 may have the following logical components: a context aggregator 402, an access policy enforcer 404, an access policy store 406, a provider handler 408, a context store 410 and context schema 412. Analyzer 204 may have the following logical components: a context analyzer 414, an analysis rules access 416, an analysis rules store 418, script-based rules 420 and pre-compiled rules supplied in binary form 422. The directed arrows of Figure 4 illustrate dependencies among the logical components, according to one embodiment of the invention.

For example, referring to Figure 4, provider handler 408 and context analyzer 414 depend on context aggregator 402 in order to add new context information in the context repository. Context aggregator 402 depends on context store 410 to make use of the database storage and retrieval facilities.

Context schema 412 consists of classes and associations, which represent fundamental elements of the context information. The schema classes form the basis of the processing actions that occur in context engine 102. The main classes of the context schema include context identifiers, context items, context groups and context data. An embodiment of the context schema is described below in more detail with reference to Figure 5. It is important to note that all components illustrated in Figure 4 depend on or make use of context schema 412.

In embodiments, context store 410 maintains in-memory instances (e.g., caches) of context information for efficient access by the other logical components in context engine 102. Here, the recently-used and frequently-used context information is typically maintained in the caches. Context store 410 is typically expected to make use of a non-volatile storage mechanism (e.g., a relational database (RDBMS) engine) to store context information, although embodiments of the invention are not limited in this regard. For example, there could be instances where non-volatile storage is not needed by context engine 102.

Access policy store 406 and access policy enforcer 404 are responsible for fulfilling the requests of clients 108 for context information. In embodiments, primary responsibilities may include making context information available to clients 108, enforcing client-level access restrictions while exposing context information, maintaining notification requests per the client and sending notifications when the requested context items become available.

Provider handler 408 interacts with providers 106 via provider API 112 and receives context information to be used by context engine 102.

Analysis rules access 416 allows clients 108 to manage analysis rules in context engine 102. Here, clients 108 may perform various operations on analysis rules including, but not necessarily limited to, add, remove, activate, and so forth.

Context analyzer 414 may execute zero or more analysis rules when a new context item becomes available in context store 410. Analysis rules store 418 represents a repository where all analysis rules are maintained. Script-based rules 420 and/or pre-compiled rules in binary form 422 are two possible types of categories of analysis rules that are supported by context analyzer 414.

An embodiment of a context schema 500 is described next in more detail with reference to Figure 5. In an embodiment, all icons in Figure 5 are as defined by the Unified Modeling Language (UML). Referring to Figure 5, context schema 500 may include the classes of a context group 502, a context identifier 504, a context item 506 and a context data 508. The exemplary context schema 500 shown in Figure 5 is one possible schema and is not meant to limit the invention.

Context groups 502 represent a collection of one or more context identifiers 504. Context identifiers 504 represent a particular type of context information (e.g., 'temperature' type). Context items 506 each represent an instance of the context information corresponding to a context identifier 504 (e.g., a temperature reading). Typically, context engine 102 will maintain multiple context items 506 for a particular context identifier 504. Context data 508 represent one value of a context item 506. In some cases, a context item 506 is allowed to have multiple valid values at the same time. Therefore, value representation is abstracted out in this class. Each of the classes is described next in more detail.

Context identifier 504 is the core representation of a context type in context engine 102. In general, context identifier 504 specifies characteristics of a context type, including how to represent and interpret its values. Representation of additional characteristics of context type is possible and contemplated by embodiments of the invention. In embodiments, clients 108 access or ask for context item 506 by specifying a context identifier 504. Other indirect mechanisms of accessing context items 506 are also possible and contemplated by embodiments of the invention.

In embodiments, a new context identifier may be supplied by various sources. One source is via providers 106. Here, as part of the provider registration process, a provider supplies definitions for all context identifiers that it is capable of providing. Another source of new context identifier is via clients 108. Here, a client may define a new context identifier as part of specifying a new context analysis rule.

Context engine 102 may maintain a list of all context identifiers 504 known to it. As new context identifiers 504 are received from providers 106, the new context identifiers are checked against known context identifiers. In a similar manner, analyzer 204 may specify a new context identifier 504 when adding a new analyzer rule (in a script based rules 420, for example).

Referring to Figure 5, an embodiment of context identifier 504 may have four primary attributes: context-name, description, value-schema, and multi-valued. In addition, in an embodiment, the context identifier 504 has member attributes corresponding to the associations shown in the figure. Table 1 below illustrates possible descriptions for primary attributes:

**Table 1**

| | |
|---|---|
| Name | Name of the context type. For example: "temperature", "traffic", "blood-pressure", etc. |
| | A context type is usually known by its name. In an embodiment, each context identifier will have a unique name, where the name will be specific to the domain of usage, such as "ambient temperature", "body temperature", "car traffic", etc. In an embodiment, the uniqueness of context identifier may be implemented by using globally unique identifiers (GUID) as an additional attribute of the class context identifier 504. |
| Description | A user-readable description for the context-identifier. In an embodiment, this field may be represented as a string in typical programming languages. This is a pass-through field. The context engine typically does not make use of this field. |
| Value schema | Specifies how context values corresponding to current context-identifier 504 are to be represented and interpreted. |
| | Referring to Figure 5, this attribute essentially prescribes format and meaning to the value attribute of the context data class 508. As an example, in an embodiment, value of "body temperature" context may be represented as floating point number and interpreted as degrees Fahrenheit as the unit of measure. The value schema attribute may specify this as: "<Fahrenheit, Float32>" |
| | As another example, in an embodiment, value of "meeting logistics" context may be represented as a combination of multiple elements. The value schema attribute may specify this as: |
| | "<RoomNumber, string>, <start-time=time>,<end-time=time>,<attendee-count=UInt>" |
| | In an embodiment, this attribute may be implemented as an XML formatted string. |
| | In addition, value-schemas may be specified and standardized for comprehensive sets of context identifiers 504 corresponding to specific domains of usages. |
| Multi valued | Indicates whether a context item 506 corresponding to the current context-identifier 504 can possibly have multiple valid context data 508 objects at the same time. |
| | In an embodiment, this attribute may be implemented as Boolean flag. |

Context group 502 represents a collection of two or more context identifiers 504. The collection is usually meaningful in the domain in which it is created. Context groups 502 may be added or removed from context engine 102 at any time. In an embodiment, clients 108 may dynamically add and remove context group 502 objects in context engine 102. But typically, context groups 502 will be created at the time of deployment of a device in a particular domain of usage.

In embodiments, group membership is by reference, which means that context identifiers 504 that make up a context group 502 exist by themselves and deletion of a context group does not remove the member context identifiers from the context engine 102.

A context identifier 504 may be a member of (i.e., referenced in) multiple context groups 502. A context group 502 may contain other context groups (non-cyclical relations only). Also, a context group 502 may be part of multiple other context groups 502.

Referring to Figure 5, an embodiment of context group 502 may have two primary attributes: Group Name and Description. In addition, in an embodiment, the context-group 502 has member attributes corresponding to the associations shown in the figure. Table 2 below illustrates possible descriptions for primary attributes:

**Table 2**

| | |
|---|---|
| Name | Name of the context group. For example: "Weather", "Patient Vitals", etc. |
| | In an embodiment, this field may be represented as a string in typical programming languages. |
| | In an embodiment, each context group typically has a unique name. |
| Description | A user-readable description of the group. |
| | In an embodiment, this field may be represented as a string in typical programming languages. This is a pass-through field. The context engine typically does not make use of this field. |

Context item 506 represents a specific sample of context information corresponding to a context identifier 504. In the case of some context types, a context item object may have multiple valid values at the same time. Thus, the value representation for the context item class may be abstracted in a separate class context data 508.

Referring to Figure 5, an embodiment of context item 506 may have three primary attributes: Is Projected, Location and Time. In addition, in an embodiment, the context-item 506 has member attributes corresponding to the associations shown in the figure. Table 3 below illustrates possible descriptions for primary attributes:

**Table 3**

| | |
|---|---|
| Is Projected? | This Boolean field indicates whether the values associated with the context item object were actually measured by the sensor or the values were estimated by using some algorithmic computations. The estimation of value may happen with respect to a different location or a different time or both. |
| | True = data is a result of a projection (i.e. estimate for another time and/or location). |
| | False = data is a measured value. |
| Location | This field represents a location associated with a context-item object. |
| | In an embodiment, this field may be represented by a point in three-dimensional space around Earth (longitude, latitude, altitude). |
| | When 'Is Projected' = false, this is the location where the ContextData:: Value were captured. |
| | Otherwise this is the location where the ContextData:: Value were projected to be applicable. |
| | If the location is unknown or is irrelevant, this member should be a null value. |
| Time | When 'Is Projected' = false, this is the timestamp when the ContextData::Value were captured. |
| | Otherwise this is the timestamp when the ContextData::Value were projected to be applicable. |
| | If the time is unknown or is irrelevant, this member should be a null value. |

Context data 508 represents one value of a context item 506. In some embodiments, a context item is allowed to have multiple valid values at the same time. Thus, value representation is abstracted out in this class. A context-identifier 504 indicates whether its corresponding context-item 506 can possibly have multiple simultaneous valid values. In an embodiment, the possibility of multiple values is flagged by a Boolean attribute "multi-valued" of class context-identifier 504.

Referring to Figure 5, an embodiment of context data 508 may have three primary attributes: Value, Validity Duration and Certainty. Table 4 below illustrates possible descriptions for these attributes:

**Table 4**

| | |
|---|---|
| Value | This is the actual value of the context item. |
| | In an embodiment, this field may be represented as an XML formatted string. |
| | The context identifier associated with the object contains information about how to interpret this value. |
| Validity duration | The time-duration for which this data value should be considered valid. |
| | This field provides a hint regarding usefulness of the data value beyond the specified timestamp in the corresponding context-item object. After the validity duration is over, the data value may be less reliable. |
| Certainty | The likelihood (i.e. confidence) associated with the correctness of the value. |
| | In an embodiment, the certainty value may be represented as an integer in the range from -100 to +100, where: |
| | +100: value is certainly correct |
| | -100: value is certainly not possible |
| | This attribute is especially useful when context values are computed using algorithmic analysis of other context parameters. |
| | For example: |
| | (1) A provider of traffic-information context may indicate that the estimated drive time between two places is 20mins with certainty value of +70. |
| | (2) An analysis rule in analyzer 204 may determine that the values of user's current privacy-state are: "alone" with certainty of -80 and "within 5 feet of other people" with certainty of +70. |

In one embodiment, context engine 102 and/or policy engine 104 may be incorporated into any mobile device capable of performing the functionality of the invention described herein. Engines 102 and/or 104 may be implemented as part of a wired communication system, a wireless communication system, or a combination of both. In one embodiment, for example, engines 102 and/or 104 may be implemented as a mobile computing device having wireless capabilities. A mobile computing device may refer to any device having a processing system and a mobile power source or supply, such as one or more batteries, for example. In other embodiments, context platform framework 100 (e.g., context engine 102 and/or policy engine 104) may also be deployed on a stationary computing device, such as a desktop PC, a backend server, and so forth.

Examples of embodiments of a mobile computing device that may be adapted to include the functionality of the present invention include a laptop computer, ultra-laptop computer, portable computer, handheld computer, palmtop computer, personal digital assistant (PDA), cellular telephone, combination cellular telephone/PDA, smart phone, pager, one-way pager, two-way pager, messaging device, data communication device, and so forth.

Examples of such a mobile computing device also may include computers that are arranged to be worn by a person, such as a wrist computer, finger computer, ring computer, eyeglass computer, belt-clip computer, arm-band computer, shoe computers, clothing computers, and other wearable computers.

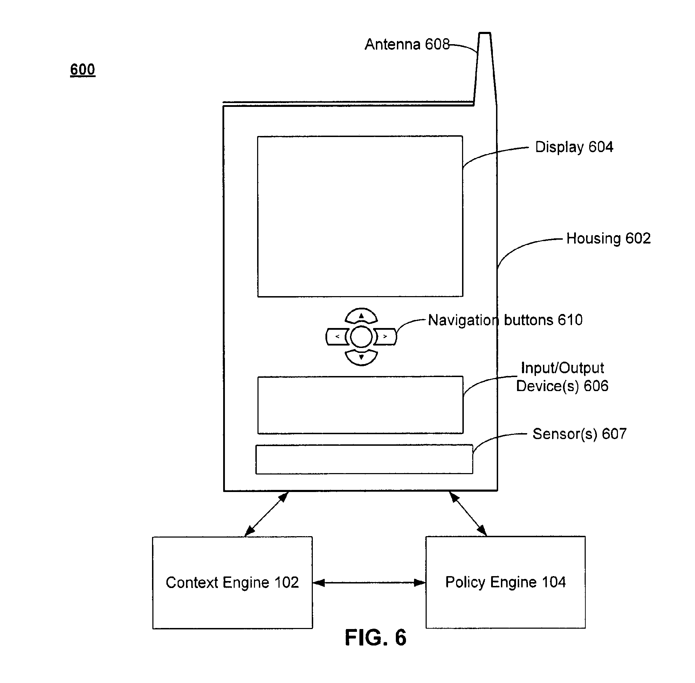

A more detailed description of an embodiment of the invention is shown in Figure 6. Referring to Figure 6, device 600 may include a housing 602, a display 604, one or more input/output devices 606, one or more sensors 607, an antenna 608, navigation buttons 610, context engine 102 and policy engine 104.

Context engine 102 and/or policy engine 104 may be directly integrated into device 600 or may be coupled to device 600 via a connection (e.g., wireless, wired or some combination of both). Note that although the functionality of engines 102 and 104 is described herein as being separated into two components, this is not meant to limit the invention. In fact, this functionality may be combined into one component or separated into three for more components. Each of the components of Figure 6 is described next in more detail.

Housing 602 may comprise any suitable housing, but typically involves a small form factor for device 600 to be easily transportable. Display 604 may comprise any suitable display unit for displaying information appropriate for the functionality of engines 102 and/or 104. Display 604 is used by embodiments of the invention to assist with input into device 600, such as customized control policy rules (as described above), and so forth.

I/O device(s) 606 may comprise any suitable I/O device for entering information into and receiving information from device 600. Here, I/O device(s) may be used to enter the data from providers 106, to send context information to clients 108, to enter customized control policy rules into policy engine 104 and analyzer 204, and so forth. Examples for I/O device(s) 606 may include touch screen interfaces, simple menus with icon selection, gestural manipulation of the device, a suitable alphanumeric keyboard, a numeric keypad, a touch pad, input keys, buttons, switches, rocker switches, a microphone, a speaker, voice recognition device and software, as well as all of the physiological sensing described above, and so forth. Information may be entered into device 600 by way of microphone. Such information may be digitized by a voice recognition device. The embodiments are not limited in this context.

Sensors 607 may comprise any suitable sensor for collecting context information to be used by context engine 102. Example sensors 607 may include one or more of the sensors described above with reference to Figure 1.

Antenna 608 is used to facilitate wireless communication with embodiments of the invention. In one embodiment, navigation buttons 610 comprise an upward navigation button, a downward navigation button, a leftward navigation button, and a rightward navigation button. Navigation buttons 610 also may comprise a select button to execute a particular function on device 600.

Operations for the above embodiments may be further described with reference to the following figures and accompanying examples. Some of the figures may include a logic flow. Although such figures presented herein may include a particular logic flow, it can be appreciated that the logic flow merely provides an example of how the general functionality as described herein can be implemented. Further, the given logic flow does not necessarily have to be executed in the order presented unless otherwise indicated. In addition, the given logic flow may be implemented by a hardware element, a software element executed by a processor, or any combination thereof.

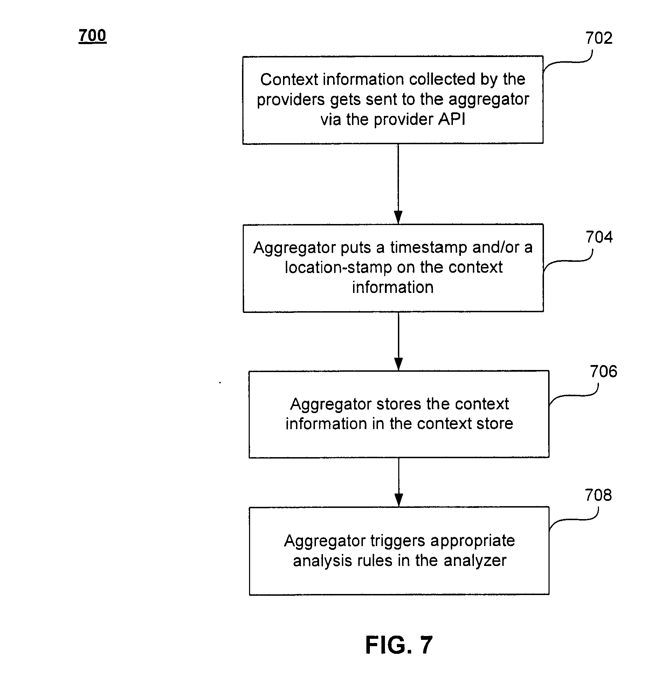

Figures 7-10 illustrate embodiments of logic flows that may be representative of the operations executed by one or more embodiments described herein, for example, the operations executed by context platform framework 100.

Figure 7 illustrates one embodiment of a logic flow 700 illustrating how context information may be provided to aggregator 202. Referring to Figure 7, context information is collected via one or more providers 106 and sent to aggregator 202 via provider API 112 (block 702).

Aggregator 202 puts a timestamp and/or location-stamp on the context information (block 704).

Aggregator 202 stores the context information in a context store (block 706).

Aggregator 202 triggers appropriate analysis rules in analyzer 204 (block 708).

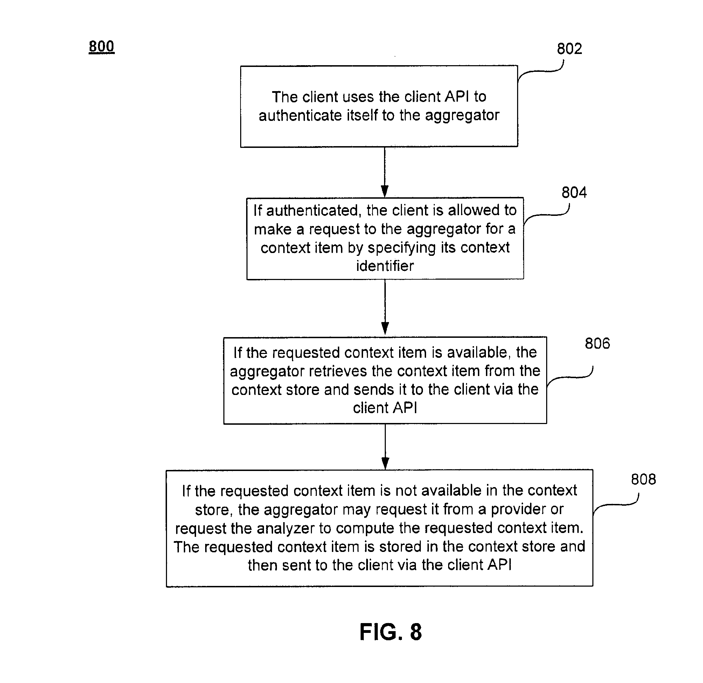

Figure 8 illustrates one embodiment of a logic flow 800 illustrating how a client 108 may access context data in aggregator 202. Referring to Figure 8, client 108 uses the client API 114 to authenticate itself to aggregator 202 (block 802).

If client 108 is authenticated, client 108 is allowed to make a request to aggregator 202 for a context item 506 by specifying its context identifier 504 (block 804).

If the requested context item is available in a context store, aggregator 202 retrieves the context item from the context store and sends it to client 108 via client API 114 (block 806).

If the requested context item is not available in the context store, then aggregator 202 may request it from one or more of providers 106. Aggregator 202 may also request analyzer 204 to computer context item 506. Context item 506 is stored in the context store and sent to client 108 via client API 114 (block 808).

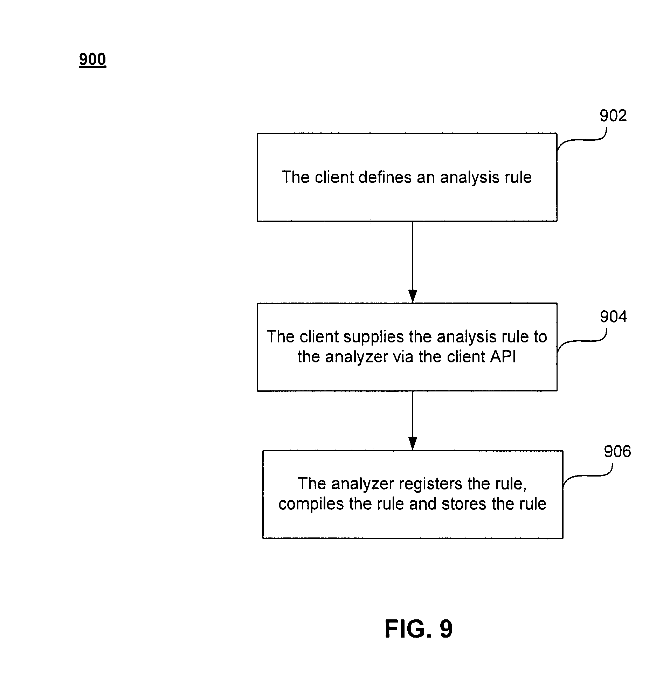

Figure 9 illustrates one embodiment of a logic flow 900 illustrating how a client 108 may provide an analysis rule to analyzer 204. Referring to Figure 9, client 108 defines an analysis rule (block 902).

Client 108 supplies the analysis rule to analyzer 204 via client API 114 (block 904).

Analyzer 204 registers the analysis rule, compiles the analysis rule and stores the analysis rule in an analysis rules store (block 906).

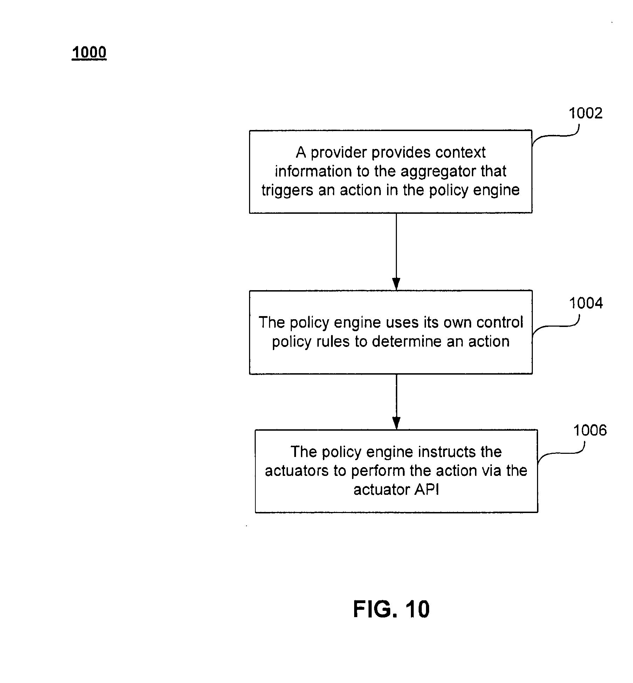

Figure 10 illustrates one embodiment of a logic flow 1000 of the operation of policy engine 104. Referring to Figure 10, a provider 106 provides context information to aggregator 202 that triggers an action in policy engine 104 (block 1002).

Policy engine 104 uses its own control policy rules to determine an action (block 1004).

Policy engine 104 instructs an actuator 110 to perform the action via the actuator API 116 (block 1006).

Various embodiments may be implemented using hardware elements, software elements, or a combination of both. Examples of hardware elements may include processors, microprocessors, circuits, circuit elements (e.g., transistors, resistors, capacitors, inductors, and so forth), integrated circuits, application specific integrated circuits (ASIC), programmable logic devices (PLD), digital signal processors (DSP), field programmable gate array (FPGA), logic gates, registers, semiconductor device, chips, microchips, chip sets, and so forth. Examples of software may include software components, programs, applications, computer programs, application programs, system programs, machine programs, operating system software, middleware, firmware, software modules, routines, subroutines, functions, methods, procedures, software interfaces, application program interfaces (API), instruction sets, computing code, computer code, code segments, computer code segments, words, values, symbols, or any combination thereof. Determining whether an embodiment is implemented using hardware elements and/or software elements may vary in accordance with any number of factors, such as desired computational rate, power levels, heat tolerances, processing cycle budget, input data rates, output data rates, memory resources, data bus speeds and other design or performance constraints.

Some embodiments may be described using the expression "coupled" and "connected" along with their derivatives. These terms are not intended as synonyms for each other. For example, some embodiments may be described using the terms "connected" and/or "coupled" to indicate that two or more elements are in direct physical or electrical contact with each other. The term "coupled," however, may also mean that two or more elements are not in direct contact with each other, but yet still co-operate or interact with each other.

Some embodiments may be implemented, for example, using a machine-readable or computer-readable medium or article which may store an instruction or a set of instructions that, if executed by a machine, may cause the machine to perform a method and/or operations in accordance with the embodiments. Such a machine may include, for example, any suitable processing platform, computing platform, computing device, processing device, computing system, processing system, computer, processor, or the like, and may be implemented using any suitable combination of hardware and/or software. The machine-readable medium or article may include, for example, any suitable type of memory unit, memory device, memory article, memory medium, storage device, storage article, storage medium and/or storage unit, for example, memory, removable or non-removable media, erasable or non-erasable media, writeable or re-writeable media, digital or analog media, hard disk, floppy disk, Compact Disk Read Only Memory (CD-ROM), Compact Disk Recordable (CD-R), Compact Disk Rewriteable (CD-RW), optical disk, magnetic media, magneto-optical media, removable memory cards or disks, various types of Digital Versatile Disk (DVD), a tape, a cassette, or the like. The instructions may include any suitable type of code, such as source code, compiled code, interpreted code, executable code, static code, dynamic code, encrypted code, and the like, implemented using any suitable high-level, low-level, object-oriented, visual, compiled and/or interpreted programming language.

Unless specifically stated otherwise, it may be appreciated that terms such as "processing," "computing," "calculating," "determining," or the like, refer to the action and/or processes of a computer or computing system, or similar electronic computing device, that manipulates and/or transforms data represented as physical quantities (e.g., electronic) within the computing system's registers and/or memories into other data similarly represented as physical quantities within the computing system's memories, registers or other such information storage, transmission or display devices. The embodiments are not limited in this context.

Numerous specific details have been set forth herein to provide a thorough understanding of the embodiments. It will be understood by those skilled in the art, however, that the embodiments may be practiced without these specific details. In other instances, well-known operations, components and circuits have not been described in detail so as not to obscure the embodiments. It can be appreciated that the specific structural and functional details disclosed herein may be representative and do not necessarily limit the scope of the embodiments.

Although the subject matter has been described in language specific to structural features and/or methodological acts, it is to be understood that the subject matter defined in the appended claims is not necessarily limited to the specific features or acts described above. Rather, the specific features and acts described above are disclosed as example forms of implementing the claims.

## Claims

1. A method, comprising:
receiving context information from a plurality of providers via a provider application program interface (API);
aggregating the received context information;
analyzing the received context information and aggregated context information to generate computed context information; and
based on the aggregated context information, the computed context information and one or more analysis rules, triggering an action for at least one client of a plurality of clients,
wherein communication with the plurality of clients is accomplished via a client API.

2. The method of claim 1, wherein each of the plurality of clients and each of the plurality of providers is domain-specific.

3. The method of claim 1, wherein the analysis rules may be customized by the plurality of clients.

4. The method of claim 1, wherein aggregating the received context information decouples the received context information from its source.

5. The method of claim 1, wherein the received context information is aggregated based on criteria supplied by the plurality of clients.

6. A system, comprising:
an aggregator to receive context information from a plurality of providers via a provider application program interface (API) and wherein the aggregator to aggregate the received context information; and
an analyzer to analyze the received context information and the aggregated context information to generate computed context information,
wherein based on the aggregated context information, the computed context information and one or more analysis rules, at least one of the aggregator and the analyzer to trigger an action for at least one client of a plurality of clients, wherein communication with the plurality of clients is accomplished via a client API.

7. The system of claim 6, wherein each of the plurality of clients and each of the plurality of providers is domain-specific.

8. The system of claim 6, wherein the analysis rules may be customized by the plurality of clients.

9. The system of claim 6, wherein aggregating the received context information decouples the received context information from its source.

10. The system of claim 6, wherein the received context information is aggregated based on criteria supplied by the plurality of clients.

11. A machine-readable storage medium containing instructions which, when executed by a processing system, cause the processing system to perform a method, the method comprising:
receiving context information from a plurality of providers via a provider application program interface (API);
aggregating the received context information;
analyzing the received context information and aggregated context information to generate computed context information; and
based on the aggregated context information, the computed context information and one or more analysis rules, triggering an action for at least one client of a plurality of clients,
wherein communication with the plurality of clients is accomplished via a client API.

12. The machine-readable storage medium of claim 11, wherein each of the plurality of clients and each of the plurality of providers is domain-specific.

13. The machine-readable storage medium of claim 11, wherein the analysis rules may be customized by the plurality of clients.

14. The machine-readable storage medium of claim 11, wherein aggregating the received context information decouples the received context information from its source.

15. The machine-readable storage medium of claim 11, wherein the received context information is aggregated based on criteria supplied by the plurality of clients.
